# EUROPEAN PATENT APPLICATION

(11) **EP 3 777 536 A1**
(43) Date of publication of application: **17.02.2021**
(21) Application number: 20177593.9
(22) Date of filing: 10.06.2009
(51) Int. Cl.: A01N 45/00, A61K 31/56, A61K 9/08, A61P 27/02, A61P 27/06, A61P 29/00, A61P 31/04, A61P 37/08, A61K 31/79

(54) **POVIDONE IODINE, A NOVEL ALTERNATIVE PRESERVATIVE FOR OPHTHALMIC COMPOSITIONS**

(30) Priority: 12.06.2008 US 129222 P; 15.12.2008 US 201854 P
(62) Divisional of application: 09762915.8
(71) Applicant: Takeda Pharmaceutical Company Limited, Osaka-shi, Osaka (JP)
(72) Inventor: CAPRIOTTI,, Joseph, A., New York,, 10025 (US); LIANG, Bo, East Brunswick,, NJ New Jersey 08816 (US); SAMSON, C.,, Michael, New York,, 10016 (US); STEIN,, Jason, New York,, 10162 (US); WEISER,, Michael, New York, (US)
(74) Representative: Carpmaels & Ransford LLP

(57) **Abstract**

The invention includes a preserved ophthalmic preparation comprising povidone- iodine (PVP-I) at a concentration sufficient to preserve the ophthalmic preparation, and at least one member selected from the group consisting of a steroidal anti-inflammatory compound, a non-steroidal anti-inflammatory compound, an antibacterial compound, an anti-allergy compound, and an anti-glaucoma compound. The invention also includes adding PVP-I to an ophthalmic composition in order to preserve the composition, wherein the PVP-I is added at a concentration sufficient to preserve the composition.

## Description

### CROSS-REFERENCE TO RELATED APPLICATIONS

This application claims priority to U.S. Provisional Patent Application No. 61/129,222, filed on June 12, 2008, and to U.S. Provisional Patent Application No. 61/201,854, filed on December 15, 2008, both of which are incorporated by reference herein in their entirety.

### BACKGROUND

Preservatives are currently an integral part of ophthalmic preparations. Suitable antimicrobial preservatives are typically added to prevent multi-dose package contamination. Such agents may include benzalkonium chloride, thimerosal, chlorobutanol, methyl paraben, propyl paraben, phenylethyl alcohol, EDTA, sorbic acid, Onamer M, other agents known to those skilled in the art, or a combination thereof. Benzalkonium chloride (also referred to as BAK or BAC) is the most common preservative used in ophthalmic preparations. Typically, such preservatives are employed at a level of from 0.001% to 1.0% by weight. However, recent evidence highlight the potential corneal and conjunctival toxicity of BAK, which lead to serious medical problems such as ocular medicamentosa, decreased success of glaucoma surgery, or reduced compliance with ocular medications.

### BRIEF SUMMARY OF THE INVENTION

The invention includes a preserved ophthalmic preparation comprising povidone-iodine (PVP-I) at a concentration sufficient to preserve the ophthalmic preparation, and at least one member selected from the group consisting of a steroidal anti-inflammatory compound, a non-steroidal anti-inflammatory compound, an antibacterial compound, an anti-allergy compound, and an anti-glaucoma compound.

In an embodiment, PVP-I is present at a concentration selected from the group consisting of 0.01% to 10%, 0.1% to 2.5%, 0.2 to 1.5%, 0.3% to 1.0%. In another embodiment, PVP-I is present at a concentration of 0.5%.

In an embodiment, the PVP-I concentration is measured on a weight/weight basis with respect to the overall preparation. In another embodiment, the PVP-I concentration is measured on a weight/volume basis with respect to the overall preparation.

In an embodiment, a preparation includes an anti-inflammatory compound selected from the group consisting of ketotifen fumarate, diclofenac sodium, nepafenac, bromfenac, flurbiprofen sodium, suprofen, celecoxib, naproxen, rofecoxib, and any combination thereof.

In an embodiment, a preparation includes an anti-inflammatory compound selected from the group consisting of dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, and any combination thereof.

In an embodiment, a preparation further includes an antimicrobial preservative selected from the group consisting of benzalkonium chloride, thimerosal, chlorobutanol, methyl paraben, propyl paraben, phenylethyl alcohol, EDTA, sorbic acid, Onamer M and any combination thereof.

In an embodiment, a preparation further includes a viscosity increasing agent. In an embodiment, a viscosity increasing agent is selected from the group consisting of polyvinyl alcohol, polyvinylpyrrolidone, methyl cellulose, hydroxypropylmethylcellulose, hydroxyethylcellulose, carboxymethylcellulose, hydroxypropylcellulose, and any combination thereof.

In an embodiment, a preparation further com includes prises at least one artificial tears-based lubricant.

The invention also includes a method of preserving an ophthalmic preparation, the method comprising adding to an ophthalmic preparation PVP-I in an amount sufficient to preserve the ophthalmic preparation. In an embodiment, the method includes adding PVP-I to exist at a concentration selected from the group consisting of 0.01% to 10%, 0.1% to 2.5%, 0.2 to 1.5%, and 0.3% to 1.0%. In an embodiment, PVP-I is present at a concentration of 0.5%.

### DETAILED DESCRIPTION

As used herein, the term "about" means plus or minus 10% of a referenced value, inclusive of the value.

Ranges set forth herein are intended to include every value between the two referenced endpoints, inclusive of the endpoint values.

Herein, it is now shown that povidone iodine can serve as a preservative for variety of pharmaceutical compositions. In an aspect of the invention, povidone iodine is shown to be a preservative for ophthalmic preparations. Therefore, in an embodiment, the invention provides a preserved ophthalmic composition comprising a povidone iodine composition.

In an embodiment, povidone iodine functions as at least a preservative for variety of pharmaceutical compositions. In an embodiment, povidone iodine is a preservative in an ophthalmic composition. The concentration of povidone-iodine as a preservative in ophthalmic compositions can range from 0.01% - 10% (weight/weight or weight/volume), and all concentrations in between. In an embodiment, the povidone-iodine concentration is between 0.1% and 2.5%, in another embodiment, between 0.2 and 1.5%, and in yet another embodiment, between 0.3% and 1.0%. In an embodiment, the povidone-iodine concentration is about 0.5%.

In an aspect of the invention, povidone iodine provides an antimicrobial property to an ophthalmic preparation. In another aspect, povidone iodine provides an ophthalmic preparation with one or more non-antimicrobial preservative properties (e.g., antioxidant).

In an embodiment, the invention also provides povidone-iodine compositions comprising one or more components in addition to the povidone-iodine component, as set forth herein.

In an aspect, the invention provides a broad spectrum of povidone-iodine ophthalmic compositions, for example, comprising povidone iodine as a preservative to be used in cases of ocular conjunctival or corneal infection caused by mycobacteria, viruses, fungi, and amoeba. In another aspect, compositions of the invention are useful in the infectious prophylaxis of patients recovering from recent ophthalmic surgery, among other ophthalmic procedures.

In various embodiments, povidone-iodine ophthalmic compositions according to the invention include, but are not limited to the following:
1. Artificial-tears preparations comprising povidone iodine as a preservative. Artificial-tear based constituents include, but are not limited to, ophthalmically-acceptable lubricants, propylene glycol, glycerin, polyethylene glycol, dextran, blended polyvinyl alcohols, polyvinyl alcohol, polyethylene glycol, light mineral oil, hydroxypropyl methylcellulose, hypromellose, carbopol, carbomer 940 (polyacrylic acid), polyvinyl pyrrolidone, white petrolatum, soy lecithin, and sodium carboxyl methylcellulose, as well as other agents known to those skilled in the art, or any combination thereof. Typically, such constituents are employed at a level of from 0.1% to 2% by weight. In an embodiment, the constituents are 1.0% Propylene glycol, 0.3% glycerin, 2.7% blended polyvinyl alcohols, 1% Polyvinyl Alcohol, 1% Polyethylene Glycol, light mineral oil, 0.3% hydroxypropyl methylcellulose, 1.0% Soy lecithin, 0.25% or 0.5% sodium carboxyl methylcellulose. In another embodiment, the total weight of the PVP-I, artificial-tear based constituents is between 0.1% and 4.5% of the total weight of the composition.
2. Anti-inflammatory and steroid preparations comprising povidone iodine as a preservative. Non-limiting examples of suitable anti-inflammatories include: ketotifen fumarate, diclofenac sodium, nepafenac, bromfenac, flurbiprofen sodium, suprofen, celecoxib, naproxen or rofecoxib. Non-limiting examples of suitable steroids include: Dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine. The anti-inflammatory and steroid constituents are typically used at a concentration of 0.01% to 2.0% by weight of the total composition. In an aspect, about 0.1% dexamethasone is used in a preserved ophthalmic preparation.
3. BAK-free ophthalmic compositions to treat glaucoma comprising povidone iodine as a preservative. Non-limiting examples of suitable glaucoma medicines include: Beta Blockers (levobunolol, timolol hemihydrate, betaxolol hydrochloride, timolol maleate, and related salts thereof); prostaglandin analogs (for example bimatoprost, travoprost, Latanoprost); Alpha Agonists (brimonidine, Iopidine, apraclonidine); Carbonic Anhydrase Inhibitors (brinzolamide, dorzolamide). Such constituents are used at concentrations typically used in the art.
4. Antibiotic /Antimicrobial ophthalmics comprising povidone iodine as a preservative. Non-limiting examples of suitable include fluoroquinolones (ciprofloxacin, levofloxacin, ofloxacin, moxifloxacin, gatifloxacin, etc...); Aminoglycosides (tobramycin, gentamicin, neomycin, etc...); Polymyxin B Combinations (polymyxin B/trimethoprim, Polysporin polymyxin B/bacitracin Neosporin polymyxin B/neomycin/ gramicidin, etc.) and other antibiotics (azithromycin, ilotycin, erythromycin, bacitracin, etc...). Typically, such antibiotic and antimicrobial constituents are employed at a level of from 0.001 % to 1.0% by weight of the total composition.
5. Anti-Allergic preparations comprising povidone iodine as a preservative. Non-limiting examples of suitable components include epinastine, emedastine difumarate azelastine hydrochloride, olopatadine hydrochloride, olopatadine, ketotifen fumarate, pemirolast potassium, nedocromil, lodoxamide, cromolyn and cromolyn salts. Such constituents are used at concentrations typically used in the art.
6. Multiple preservative ophthalmic preparations comprising povidone iodine as a preservative. Non-limiting examples of suitable components include an antimicrobial preservative. In an embodiment, an antimicrobial preservative is selected from the group consisting of benzalkonium chloride, thimerosal, chlorobutanol, methyl paraben, propyl paraben, phenylethyl alcohol, EDTA, sorbic acid, Onamer M and combinations thereof. Such constituents are used at concentrations typically used in the art.

Additionally, non-limiting examples of suitable excipients for povidone-iodine compositions of the invention include co-solvents/surfactants, viscosity-altering agents, and/or bioadhesive agents. Such constituents are used at concentrations typically used in the art.

In an aspect, the compositions of the invention may optionally include a co-solvent or surfactant. In an embodiment, a co-solvent may be the same or different than a surfactant. In an embodiment, the solubility of the components of the compositions may be enhanced by inclusion of a surfactant or appropriate co-solvent in the composition. Such co-solvents/surfactants include, but are not limited to, polysorbate-20, -60, and -80, polyoxyethylene /polyoxypropylene surfactants (e.g. Pluronic F-68, F-84 and P-103), cyclodextrin, tyloxapol, PEG 35 Caster oil (Cremophor EL), polyoxyl 40 Stearate (Myrj 52), as well as other agents known to those skilled in the art, or any combination thereof. Typically, such co-solvents are employed at a level of from 0.01% to 2% by weight.

In another aspect, the compositions of the invention may optionally comprise an optional viscosity-increasing or viscosity-decreasing agent. Viscosity increased above that of simple aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity-enhancing agents include, but are not limited to, polyvinyl alcohol, polyvinylpyrrolidone, methyl cellulose, hydroxypropylmethylcellulose, hydroxyethylcellulose, carboxymethylcellulose, hydroxypropylcellulose, other agents known to those skilled in the art, and/or any combination thereof. Such agents are typically employed at a level of from 0.01% to 2% by weight.

In another aspect, bioadhesive agents may comprise the compositions, in order to increase the retention time of the drug gradient over a biological substrate. The bioadhesive agents include, but are not limited to: polyvinylpyrrolidone (PVP), xanthan gum, locust bean gum, acacia gum, hydroxypropyl methylcellulose (HPMC), sodium alginate, pectin, gelatin, carbomer, polyvinylalcohol, gellan gum, tragacanth, acacia, and sodium carboxymethyl cellulose, as well as other agents known to those skilled in the art, or any combination thereof.

Furthermore, the compositions can be combined with an effective amount of a chemical agent to provide a cooling sensation to relieve mild ocular irritation, enhance ocular comfort, provide a refreshing effect, and improved sensation, when the povidone-iodine solution is applied to the eyes. Such an agent encompasses various chemicals and chemical classes, including, but not limited to, cooling agents such as menthol, menthol derivatives including methone glycerin acetyl and menthyl esters, carboxamides, menthane glycerol ketals, alkyl substituted ureas, sulfonamides, terpene analogs, furanones, and phosphine oxides; or camphor, and borneol.

The povidone-iodine-comprising composition may be in the form of a solution, a suspension, an emulsion, an ointment, a cream, a gel, or a controlled-release/sustain-release vehicle. For example, the composition may be in the form of a contact lens solution, eyewash, eyedrop, and the like.

In any of the compositions of this disclosure for topical administration, such as topical administration to the eye, the mixtures can be formulated as aqueous solutions at a pH in the range of 3.5 to 6.5. It will be understood that a range listed herein is intended to encompass the upper and lower bounds of the range, inclusively. In an embodiment, the pH is in the range of 4 to 5. This pH range may be achieved by the addition of acids/bases to the solution. In another embodiment, the pH is in the range of 3 to 7.

The invention also provides methods of using a povidone-iodine-comprising composition. In an embodiment, the dose volume administered to a subject may be between about 10 microliters and about 200 microliters, in another embodiment, between about 20 microliters and about 100 microliters, and in another embodiment, between about 50 microliters and about 80 microliters, and in another embodiment, about one drop per eye. In an aspect, one drop may be between about 50 and about 80 microliters.

In an embodiment, administration frequency may be anywhere in the range of 1 to 100 times a day. In another embodiment, administration frequency may be between 2 and 24 times a day. In another embodiment, administration frequency may be between 2 and 4 times a day. While the precise regimen can be identified by the skilled artisan, the composition may be topically applied by placing one drop in each eye about 1 to about 24 times daily. For example, the solution may be applied 1, 2, 3, 4, 5, 6, 7, 8, 9, 10, 11, 12, 13, 14, 15, 16, 17, 18, 19, 20, 21, 22, 23, 24, 48, or 96 times a day.

### EXAMPLES

The invention is now described with reference to the following Examples. These Examples are provided for the purpose of illustration only and the invention should in no way be construed as being limited to these Examples, but rather should be construed to encompass any and all variations which become evident as a result of the teaching provided herein.

### Example 1: Antimicrobial Preservative Effectiveness Tests of PVP-I preserved artificial tears

A composition including at least one artificial tear constituent according to the invention, further comprising PVP-I, may be used as a preservative. The preservative properties of a composition of the invention, such as an ophthalmic preparation comprising PVP-I, can be tested, for example, using USP <51>, or by any method known in the art to determine the effectiveness of an antimicrobial preservative. PVP-I solutions having concentrations of about 0.36% by weight are prepared as set forth herein. By way of a non-limiting example, a PVP-I containing solution is prepared using about 0.36% PVP-I, by weight, as desired in the final product, and combining the PVP-I with 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to about 4.0, and enough purified water to bring the total volume up to 100% total.

The test solutions are challenged with known numbers of standard laboratory strains selected. At periodic intervals: 0, 7, 14, and 28 days, samples are removed and assayed to determine survival.

### Example 2: Antimicrobial Preservative Effectiveness Tests of PVP-I preserved 0.1% dexamethasone suspension

The preservative properties of a composition of the invention, such as an ophthalmic preparation comprising PVP-I, can be tested, for example, using USP <51>, or by any method known in the art to determine the effectiveness of an antimicrobial preservative. PVP-I solutions having concentrations of about 0.18% and about 0.36% by weight are prepared as set forth herein. By way of a non-limiting example, a PVP-I containing solution is prepared using 0.18%, or 0.36% PVP-I, by weight, as desired in the final product, and combining the PVP-I with 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to 4.0, and enough purified water to bring the total volume up to 100% total. The test solutions are challenged with known numbers of standard laboratory strains selected. At periodic intervals: 0, 7, 14, and 28 days, samples are removed and assayed to determine survival.

### Example 3: Antimicrobial Activity of Preserved PVP-I Solutions

By way of a non-limiting example, PVP-I solutions having concentrations of about 0.36% by weight are prepared as set forth herein. By way of a non-limiting example, a PVP-I containing solution is prepared using 0.36% PVP-I, by weight, as desired in the final product, and combining the PVP-I with 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to 4.0, and enough purified water to bring the total volume up to 100% total. These solutions are then tested for *in-vitro* microbiological activity. Microbiological activity can be tested for antimicrobial activity against, for example, bacteria found in the mouth (*P. gingivalis*)*,* or against other bacteria. In another example, killing time tests are conducted with a series of log phase cultures of gram negative and gram positive organisms including Gentamicin resistant *Pseudomonas aeruoinosa, methicilin-resistant staph aureus, E. coli, chlamydia trachoma* and selected viruses. Controls used may include ophthalmic preparations of commercially available antimicrobial products. Bacterial samples are taken at 30 seconds, 1, 2, 5, 10 and 15 minutes and transferred into culture media containing inactivators for iodine. Similarly, virus killing time tests are sampled at one minute and transferred into inactivating media. The results obtained with the experimental samples are compared with the control samples to assess the level of antimicrobial activity of a composition of the invention.

### Example 4: Preparation of PVP-I preserved 0.3% tobramycin and 0.1% dexamethasone suspension

BAK-free Tobradex® ophthalmic suspension preserved with PVP-I concentration ranging from about 0.36% to about 0.6% by weight is prepared as set forth herein. By way of a non-limiting example, a composition is prepared using 0.36%, 0.48%, or 0.6% PVP-I, by weight, as desired in the final product, and combining with 0.3% tobramycin, 0.1% dexamethasone, 0.01% edentate disodium, 1.2% sodium sulfate, 0.25% hydroxyethylcellulose, 0.5% tyloxapol, 0.35% sodium chloride to adjust osmolality within 300-350 mOsm/kg, sodium hydroxide and/or sulfuric acid to adjust the pH to about 4.0, and enough purified water to bring the total volume up to 100% total.

### Example 5: Preparation of PVP-1 preserved 0.3% tobramycin solution

BAK-free tobramycin ophthalmic solution preserved with PVP-I in a concentration range of about 0.36% to about 0.6% by weight is prepared as set forth herein. By way of a non-limiting example, an composition is prepared using 0.36%, 0.48%, or 0.6% PVP-I, by weight, as desired in the final product, and combining with 0.3% tobramycin, boric acid, sodium sulfate, sodium chloride, tyloxapol, sodium hydroxide and/or sulfuric acid (to adjust pH) and purified water.

### Example 6: Preparation of PVP-I preserved 0.5% moxifloxacin hydrochloride ophthalmic solution

BAK-free moxifloxacin hydrochloride ophthalmic solution preserved with PVP-I in a concentration range of about 0.36% to about 0.6% by weight is prepared as set forth herein. By way of a non-limiting example, a composition is prepared using 0.36%, 0.48%, or 0.6% PVP-I, by weight, as desired in the final product, and combining with 0.5% moxifloxacin hydrochloride, boric acid, sodium chloride, and purified water. In an embodiment, a preserved ophthalmic solution comprises hydrochloric acid and/or sodium hydroxide to adjust pH.

### Example 7: Preparation of PVP-I preserved 1.0% prednisolone acetate suspension

BAK-free prednisolone acetate suspension preserved with PVP-I in a concentration range of about 0.36% to about 0.6% by weight is prepared as set forth herein. By way of a non-limiting example, a composition is prepared using 0.36%, 0.48%, or 0.6% PVP-I, by weight, as desired in the final product, and combining with 1.0% prednisolone acetate, boric acid, edetate disodium, hypromellose, polysorbate 80, sodium bisulfite, sodium citrate, sodium chloride, and purified water.

The invention has been described herein by reference to certain embodiments. However, as variations thereof will become apparent to those skilled in the art, when armed with the disclosure set forth herein, the invention is not to be considered as limited thereto. All patents, patent applications, and references cited herein are hereby incorporated by reference in their entirety.

A non-exhaustive list of embodiments of the disclosure is set out in the
following numbered aspects.
1. A preserved ophthalmic preparation comprising:
   a. povidone-iodine (PVP-I) at a concentration sufficient to preserve said ophthalmic preparation, and
   b. at least one member selected from the group consisting of a steroidal anti-inflammatory compound, a non-steroidal anti-inflammatory compound, an antibacterial compound, an anti-allergy compound, and an anti-glaucoma compound.
2. The ophthalmic preparation of aspect 1, wherein the PVP-I is present at a concentration selected from the group consisting of 0.01% to 10%, 0.1% to 2.5%, 0.2 to 1.5%, 0.3% to 1.0%.
3. The ophthalmic preparation of aspect 1, wherein the PVP-I is present at a concentration of 0.5%.
4. The ophthalmic preparation of aspect 2, wherein the PVP-I concentration is measured on a weight/weight basis with respect to the overall preparation.
5. The ophthalmic preparation of aspect 2, wherein the PVP-I concentration is measured on a weight/volume basis with respect to the overall preparation.
6. The ophthalmic preparation of aspect 1, wherein said anti-inflammatory compound is selected from the group consisting of ketotifen fumarate, diclofenac sodium, nepafenac, bromfenac, flurbiprofen sodium, suprofen, celecoxib, naproxen, rofecoxib, and any combination thereof.
7. The ophthalmic preparation of aspect 1, wherein said anti-inflammatory compound is selected from the group consisting of dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, and any combination thereof.
8. The ophthalmic preparation of aspect 1, wherein said preparation further comprises an antimicrobial preservative selected from the group consisting of benzalkonium chloride, thimerosal, chlorobutanol, methyl paraben, propyl paraben, phenylethyl alcohol, EDTA, sorbic acid, Onamer M and any combination thereof.
9. The ophthalmic preparation of aspect 1 wherein said preparation further comprises a viscosity increasing agent.
10. The ophthalmic preparation of aspect 9 wherein said viscosity increasing agent is selected from the group consisting of polyvinyl alcohol, polyvinylpyrrolidone, methyl cellulose, hydroxypropylmethylcellulose, hydroxyethylcellulose, carboxymethylcellulose, hydroxypropylcellulose, and any combination thereof.
11. The ophthalmic preparation of aspect 1, wherein the preparation comprises at least one artificial tears-based lubricant.
12. A method of preserving an ophthalmic preparation comprising adding to said ophthalmic preparation PVP-I in an amount sufficient to preserve said ophthalmic preparation.
13. The method of aspect 12, wherein said PVP-I is present at a concentration selected from the group consisting of 0.01 % to 10%, 0.1 % to 2.5%, 0.2 to 1.5%, 0.3% to 1.0%.
14. The method of aspect 12, wherein the PVP-I is present at a concentration of 0.5%.
15. The method of aspect 12, wherein the PVP-I concentration is measured on a weight/weight basis with respect to the overall preparation.
16. The method of aspect 12, wherein the PVP-I concentration is measured on a weight/volume basis with respect to the overall preparation.

## Claims

1. A stable ophthalmic composition suitable for topical administration to the eye, comprising a mixture of:
a. povidone-iodine (PVP-I); and
b. a steroidal anti-inflammatory compound,
and wherein the pH of the composition is in the range of 3 to 4.

2. The ophthalmic composition of claim 1, wherein the PVP-I is present at a concentration from 0.01% to 10%.

3. The ophthalmic composition of claim 1, wherein the PVP-I is present at a concentration 0.1% to 2.5%.

4. The ophthalmic composition of claim 1, wherein the PVP-I is present at a concentration of 0.5%.

5. The ophthalmic composition of any one of claims 2 to 4, wherein the PVP-I concentration is measured on a weight/weight basis with respect to the overall preparation.

6. The ophthalmic composition of any one of claims 2 to 4, wherein the PVP-I concentration is measured on a weight/volume basis with respect to the overall preparation.

7. The ophthalmic composition of any one of claims 1 to 6, wherein said composition further comprises an antimicrobial preservative, preferably selected from the group consisting of benzalkonium chloride, thimerosal, chlorobutanol, methyl paraben, propyl paraben, phenylethyl alcohol, EDTA, sorbic acid, Onamer M and any combination thereof.

8. The ophthalmic composition of any one of claims 1 to 7, wherein said composition further comprises a viscosity increasing agent, preferably selected from the group consisting of polyvinyl alcohol, polyvinylpyrrolidone, methyl cellulose, hydroxypropylmethylcellulose, hydroxyethylcellulose, carboxymethylcellulose, hydroxypropylcellulose, and any combination thereof.

9. The ophthalmic composition of any one of claims 1 to 8, wherein the composition comprises at least one artificial tears-based lubricant.

10. The ophthalmic composition of any one of claims 1 to 9, wherein said steroid is present at a concentration between 0.01 and 2%, preferably at a concentration of 0.1%.

11. The ophthalmic composition of any one of claims 1 to 10, wherein said composition further comprises a co-solvent/surfactant, preferably selected from the group consisting of polysorbate 20, polysorbate 60, polysorbate 80, Pluronic F-68, Pluronic F-84, Pluronic P-103, cyclodextrin, tyloxapol and a combination thereof.

12. The ophthalmic composition of claim 11, wherein said co-solvent/surfactant is at a concentration of about 0.01% to 2% by weight in said composition.

13. The ophthalmic composition of any one of claims 1 to 12, wherein the steroidal anti-inflammatory compound is dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate or lodoxamide tromethamine, and any combination thereof.

14. The ophthalmic composition of any one of claims 1 to 13, wherein said pH is pH 4.
